# EUROPEAN PATENT SPECIFICATION

(11) **EP 2 116 183 B1**
(45) Date of publication and mention of the grant of the patent: **01.02.2012**
(21) Application number: 08155801.7
(22) Date of filing: 07.05.2008
(51) Int. Cl.: A61B 5/024, A61B 5/0402, A61B 5/053

(54) **Robust opto-electrical ear located cardiovascular monitoring device**
Robustes optoelektrisches im Ohr positioniertes kardiovaskuläres Überwachungsgerät
Dispositif de surveillance cardiovasculaire opto-électrique robuste localisé sur l'oreille

(43) Date of publication of application: 11.11.2009
(73) Proprietor: CSEM Centre Suisse d'Electronique et de Microtechnique SA, 2002 Neuchâtel (CH)
(72) Inventor: Vetter, Rolf, 1116 Cottens (CH)
(74) Representative: P&TS SA (AG, Ltd.)

(56) References cited:
- WO-A-2006/067690
- WO-A-2007/144776
- US-A- 4 860 759
- US-A1- 2003 233 051

## Description

### Field of the invention

The present invention relates to a portable cardiovascular monitoring device providing a heart rate estimation with improved robustness and reliability.

### Description of related art

Portable cardiovascular monitoring devices are classically composed of a processing device and an external probe such as electronic stethoscope, optical measure at ear lobe, chest belt for electrocardiogram (ECG) based measurement, etc. The use of an external probe is often considered as a reduction of the user's comfort. ECG-based pulse rate detecting devices using external electrode probes are for instance disclosed in documents US4108166, US6018677, US 6149602 and WO0051680.

More recently, measuring techniques based on so-called photoplethysmography (PPG) have been proposed. The PPG measurement system classically consists of a source of radiant optical energy and at least one detector for detecting the intensity of the radiant optical energy after propagation through the human body tissue. Infra-red light is predominantly used since it is relatively well absorbed in blood and weakly absorbed in body tissue. Since light is highly scattered in tissue, a detector positioned on the surface of the skin can measure reflections (or transmissions) from a range of depths. Any change in blood volume induced, for example, by the periodic cardiovascular pulse wave can be measured with a reasonable contrast by the changing optical absorption of radiant optical energy the blood volume induces. This effect will be averaged over many arteries and veins. Data processing means can then be used for extracting bodily parameters such as heart rate or oxygen concentration in the blood. The principal advantage of PPG measurement resides in the fact that it is entirely non-invasive and can be applied to any blood bearing tissue, typically a finger, ear lobe, nose and, in some instances, wrist.

PPG has widely been used for measuring arterial oxygen saturation known as pulse oximetry. PPG-based oximetry sensing devices employing sensors which are typically in contact with the user's finger or nail are for instance disclosed in documents US5237994, US5645060, US5662106, US5934277, US6018673, WO9952420, WO9962399 and WO0125802. PPG-based oximetry and heart rate estimating devices intended to be worn on or around other parts of the human body such as the wrist or ear, are also known, for instance from documents US5807267 and WO9714357.

One of the main problems of PPG measurements is corruption of the useful signal by ambient light and other electromagnetic radiations (so-called light artifacts) and by voluntary or involuntary subject movement (so-called motion artifacts). These artifacts lead to erroneous interpretation of PPG signals and degrade the accuracy and reliability of PPG-based algorithms for the estimation of cardiovascular parameters as, for example, the heart rate.

Processing of ambient light artifacts is not critical because the influence of ambient light can be measured using multiplexing techniques and an artifact free PPG signal can be restored using subtractive-type techniques. Reference can here be made to the article "Effect of motion, ambient light, and hypoperfusion on pulse oximeter function ", Trivedi N. et al., Journal of Clinical Anaesthesia, vol 9, pp. 179-183, 1997, for a description of these problems.

The processing of motion artifacts in PPG measured signals has been addressed by the present applicant in patent application EP1297784. The application discloses a signal enhancement method that exploits the information contained in a motion reference signal generated by a three-dimensional accelerometer in order to obtain a robust PPG heart rate estimation, even under intense physical activity.

Another problem with PPG measurements concerns the reliability of the measured PPG sensor signals in conditions of reduced blood flow and/or weak pulse pressure wave, namely low perfusion. Indeed, PPG systems are commonly worn on the body extremities such as fingers and ears, where low blood flow and/or weak pulse pressure wave conditions can result from hypoperfusion, due for example, to a subject being exposed to cold temperature or at rest. These conditions result in a PPG signal that has almost no discernible arterial blood pressure wave and cannot be practically exploited. The operational range of PPG-based cardiovascular monitoring devices is therefore limited to conditions with sufficient blood flow and/or perfusion that yield a reliable PPG signal.

In typical outdoors or fitness activities, a user may be exposed to cold weather, for example, when practicing during windy conditions and/or cold temperatures. The activity intensity may vary from a resting period with insufficient perfusion due to temperature related vasoconstriction to a high intensity exercise with increased peripheral perfusion due to exercise related vasodilatation. Consequently, there is a need to extend the operational range of cardiovascular monitoring in conditions where the PPG measurements are affected by a reduced blood flow or/and low perfusion.

Patent US4860759 discloses a vital signs monitor includes an ECG sensor, an oximetric finger probe, a respiration impedance sensor, a respiration strain gauge, and a blood pressure cuff with a pressure sensor. Signals generated by these sensors are processed by a microcomputer which is programmed to cross-reference data from multiple channels in order to improve accuracy. The microcomputer is programmed to select the more regular pulses from the ECG sensor and the finger probe to arrive at a better measure of heart rate. In addition, the microcomputer is programmed to use the strain gauge signal to remove artifacts from the signal generated by the impedance sensor. In this way a more artifact-free measure of respiration rate is obtained.

Patent application W02006/067690 relates to a device and method for measuring a user's heart rate. In order to provide a reliable and inexpensive heart rate measuring technique, a device and a method for measuring a user's heart rate is suggested in which the relative movement between a heart rate sensor and the user is detected to generate a correction signal for removing motion artifacts caused by a user' movement.

Patent application US2003/233051 by the present applicant discloses a portable equipment including a heart rate measuring device and a sound reproduction unit including means for supplying a signal representative of the sound reproduction and for supplying sound information to a sound transducer. The measuring device and sound transducer are mounted in an assembly to be fixed to an ear of a wearer. The sound reproduction unit includes means for substituting for and/or superimposing on said signal representative of said sound reproduction a signal generated from signals from said measuring device and representative of said heart rate.

### Brief summary of the invention

An object of the invention is therefore to propose a new system and method which overcomes at least some limitations of the prior art.

According to the invention, these objectives are achieved by means of a system and method comprising the features of the independent claims, preferred embodiments being indicated in the dependent claims and in the description.

According to the invention, these aims are achieved by means of a portable cardiovascular monitoring device comprising a first cardiovascular (CV) sensor based on a PPG technique for providing a first heart rate estimation of a user and at least a second CV sensor based on an ECG or an impedance cardiography (ICG) method for providing a second heart rate estimation. The portable cardiovascular monitoring device comprises a control device and a signal processing module for estimating the reliability of the first and at least second heart rate estimations. An estimation of a single robust user's heart rate is then obtained by combining the different heart rate estimations according to their reliability indicators or by selecting the heart rate estimations having the highest reliability indicator.

In an embodiment of the invention, the CV sensors are placed in one or two ear-located devices worn in on or the two user's ears.

In another embodiment, the reliability determination of the heart rate estimations is based on one or more characteristics of CV signals and/or heart rate estimations such as the signal amplitude or signal-to-noise ratio and the variability of the estimated heart rates. The different characteristics can be merged through a fuzzy logic method or a threshold level method.

In yet another embodiment, the reliability determination of the heart rate estimation is based on the most likely temporal position of each heart rate pulses in CV signals received from the different CV sensors.

In yet another embodiment, the reliability determination is based on characteristics of the signal generated by the motion sensor comprised in the ear located device.

In yet another embodiment, the unreliable CV sensor signals are switched off.

The present invention allows the extension of the operational range of cardiovascular monitoring in conditions, more particularly in conditions of reduced blood flow or/and low perfusion.

The portable cardiovascular monitoring device of the invention can be used for the heart rate monitoring of a user in the context of fitness, outdoors, sportive or entertainment activities and is power consumption efficient, thereby increasing the life of a battery in the cardiovascular monitoring device.

### Brief Description of the Drawings

The invention will be better understood with the aid of the description of an embodiment given by way of example and illustrated by the figures, in which:
Fig. 1 shows a first embodiment of portable cardiovascular monitoring device comprising PPG and ECG-based CV sensors;
Fig. 2 shows portable cardiovascular monitoring device comprising the PPG and ICG-based CV sensors; and
Fig. 3 is a schematic representation of the portable cardiovascular monitoring device of the invention.

### Detailed Description of possible embodiments of the Invention

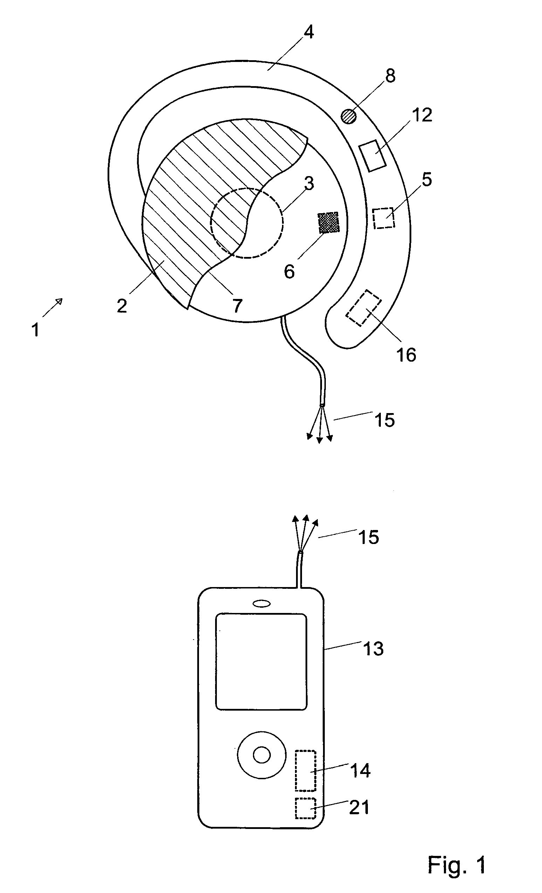

Fig. 1 shows a first embodiment of portable cardiovascular monitoring device according to the invention. The device comprises a first ear located device 1 with a generally circular casing 2 adapted to be placed inside or against the external ear (auricle) of a user in front of the external auditory meatus opening, the side of the casing 2 that can be seen in Fig. 1 facing toward the latter opening. The casing 2 of the first ear located device 1 contains a sound transducer symbolized by the dashed line circle 3. The casing 2 is fastened to a hook 4 whose free end part is adapted to hang behind the external ear of a user, as is usual for earphones used with off-the-shelf walkmans, MP players, or ipod, etc.

The first ear located device 1 is equipped with a first PPG-based CV sensor. In this first embodiment, an optical emitter 5 is placed in the hook 4 so that it directs radiation toward the casing 2. In other words, looking at Fig. 1, the emitter 5 is placed on the non visible side of the hook 4. The emitter 5 comprises one or more sources of optical radiation, preferably two infrared light emitting devices (LEDs) emitting at one or more different wavelengths. In the present configuration, these sources irradiate a portion of the cartilage of the external ear.

An optical radiation receiver 6 is disposed in the casing 2 so that it can pick up the portion of the optical radiation emitted by the emitter 5 that has passed through the thickness of the external ear. The receiver 6 comprises a plurality of optical detectors, preferably two pairs of photodiodes (not shown), whose sensitivity ranges are adjusted to the respective wavelengths of the sources. Consequently, the received radiation is a function of variations in the optical characteristics of the portion of the external ear through which the radiation has passed, which variations are caused in particular by variations in the circulation of blood in the external ear. The variations in the electrical signal delivered by the receiver 6 are therefore representative in particular of the pulsation of the blood and therefore of the heart rate.

Alternatively, the above configuration where the optical emitter 5 is placed in the casing 2 and the optical radiation receiver 6 placed in the hook 4 is also possible.

In one embodiment, the optical emitter 5 and the optical radiation receiver 6 can be placed along the hook 4, on the side which is in contact with the user's skin, for example, in a region in vicinity with the posterior auricular artery. Preferably, the receiver 6 comprises a pair light detectors disposed on each side of the emitter, formed by one light source, at a determined distance from the light source of the emitter 5, for example, about 10 mm. Other possible arrangements of the emitter 5 and receiver 6 can comprise one or several light sources and light detectors respectively. In this configuration, light from the optical emitter 5 propagates through the posterior auricular artery and is reflected towards the optical radiation receiver 6.

In another embodiment, the casing 2 comprises a part that penetrates inside the external auditory meatus or that has the shape of an earplug. Here, the optical receiver 6 is placed in the casing 2 on the side which is in contact with the external auditory meatus. The optical emitter 5 can be placed in the hook 4, on the side which is in contact with the user's skin, for example, in a region in vicinity with the superficial temporal artery. In this configuration, the light emitted from the optical emitter 5 is transmitted across the temporal artery to reach the optical radiation receiver 6.

The casing 2 can be covered with a foam material 7 (only part of which is shown) to protect the portion of the ear surrounding the external auditory meatus opening. The foam may be made of a material with flexible and adherent surface.

The electrical signals delivered by the receiver 6, called in the remaining text PPG-based CV sensor signals, are transferred to a signal acquisition module 12. The acquired signal outputted from the acquisition module 12 is transferred to a control device 13, more particularly to a signal processing module 14 through a cable 15. The acquisition module 12 and signal processing module 14 are described in more details below.

The control device 13 can be a media player such as the digital Apple iPod media player or any other handheld computing device such as a cellular telephone, personal digital assistant or other devices having an operating system. Here, the signal processing module 14 can be installed to the existing media player or handheld computing device or, alternatively, the processor of the media player or handheld computing device can be used for the sensor signal processing. Other portable devices including compact disk (CD) or versatile disks (DVD) players, radio receiver, etc., may also be used as well as a control device dedicated to the sole sensing purpose.

In the first embodiment of the invention, the portable cardiovascular monitoring device also comprises a second ECG-based CV sensor for measuring the user's heart rate.

The measurement of an ECG waveform typically involves the placement of at least two signal electrodes and a ground electrode widely spaced across the patient's body. In an embodiment shown in Fig. 1, the first ear located device 1 comprises a first ECG signal electrode 8 placed in the hook 4, for example in proximity of the optical emitter 5. A second ECG signal electrode (not shown) can be placed on the hook of a second ear located device (not shown) worn on the user's other ear or against another body part. Both signal electrodes are placed in a way to be in contact with the user's skin. A ground electrode (not shown) is preferably placed far from the ECG signal electrode, for example, in contact with the skin of the user's wrist or arm. For example, in the case the control device 13 is worn on the user's wrist or arm using an armband, the ground electrode can be located on the back of the control device 13 or armband tissue, in contact with the user's skin. The ground electrode can also be placed in any convenient location in the hook 4 or in the casing 2. Alternatively, a measuring configuration may use no ground electrode but only the first and second ECG signal electrodes, placed on the first and second ear located device, respectively. Both first and second ECG signal electrodes may also be located in the first or second ear located device.

The two ECG signal electrodes and the ground electrodes are typically metallic electrodes. Alternatively, the electrodes can be galvanized at the surface of the foam material 7. In another possible embodiment, the foam material may be embedded with electrolyte properties so as to be substantially conductive. The ECG electrodes can be connected to a comparator (not shown) for determining a difference value between the measured electric potentials at the different electrode locations.

In a second embodiment, the second CV sensor of the portable cardiovascular monitoring device is based on an impedance cardiography (ICG) method.

An impedance measurement of a patient is typically made by passing a current between two active impedance electrodes and measuring the resulting potential difference between two passive impedance electrodes. The measured impedance depends on the electrical conductivity of the tissue and fluid through which the current passes. The pulse of the bloodstream contributes to a pulsatile change in the amount and type of fluid in the current path, which is detected as a pulsatile change in the measured impedance. The measurement of pulsatile characteristics may be measured using a set of closely spaced sensors on a single housing attached to the user as it is done in an impedance plethysmography measurement (IPG) method.

Impedance measurements are typically made using a system of at least four electrodes, with the pair of active impedance electrodes being actively driven with a signal generator and the pair of passive impedance electrodes passively receiving a signal related to the active signal and the impedance of the patient.

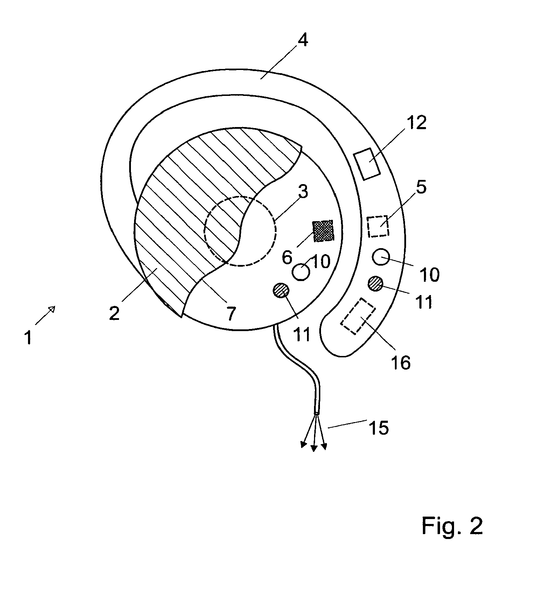

Fig. 2 illustrates an embodiment of the invention, where the first ear located device 1 comprises a pair of active impedance electrodes 10, where one of the active electrodes is disposed on the hook 4 and the other is placed on the casing 2, both electrodes being in contact with the user's skin, so that a current passes between the two active electrodes 10 through the thickness of the external ear. A potential difference arising due to the current passing between the two active electrodes 10 is measured between two passive impedance electrodes 11, also placed in contact with the user's skin, on the hook 4 and on the casing 2, respectively. Other arrangements of the active impedance electrodes are also possible. For example, one of the two active electrodes 10 can be placed on the first ear located device 1 and the other on the second ear located device, on the hook and/or on the casing 2 respectively. This latter arrangement results in a more robust CV sensor signal due to the greater bloodstream in the current path. An example of this type of impedance measurement configuration is described in patent US5178154, titled "Impedance Cardiograph and Method of Operation Utilizing Peak Aligned Ensemble Averaging".

The impedance measurement can be made in substantially the same physical location as optical measurement as shown in figure 2 or can also be made at any other suitable location. The pairs of active and passive impedance electrodes can also be aligned at the surface of the hook or of the casing 2, for example, galvanized at the surface of the foam material, in contact with the user's skin.

Other portable cardiovascular monitoring device and sensor arrangements are also possible.

In one embodiment, the optical and/or electrical CV sensors can be placed on a branch extending from the ear cushion in the neck area, in the vicinity of an artery (carotide).

In another embodiment, the portable cardiovascular monitoring device can be placed in a hearing aid system such as behind-the-ear, in-the-ear, in-the-canal, etc., hearing aid types.

In yet another embodiment, the portable cardiovascular monitoring device uses small earphones, placed outside the external auditory meatus such as the distinctive white earphones that are included with many portable music players, or placed inside the external auditory meatus, such as canalphones. Here, the optical emitter 5 and the optical radiation receiver 6 are placed on the surface of one of the earphones and the light emitted by the optical emitter 5 propagates through the tissue in the vicinity of the external auditory meatus and is reflected towards the optical radiation receiver 6. In addition, each earphone may contain one ECG signal electrode with the ground ECG electrode being placed on one of the earphone. The two pairs of active and passive impedance electrodes 10, 11 can be arranged equidistant in one of the earphone.

In yet another embodiment, the optical and electrical CV sensors are placed in a headband fitted to the head of a user. Preferably, the PPG and ICG-based CV sensors are placed at locations in the vicinity of a superficial artery such as, for example, the superficial temporal artery in the temporal region or the supraorbital or zygomatico-orbital artery in the front head area. The ECG signal electrodes can be placed at opposite locations.

It is understood that the present invention is not limited to the exemplary embodiments described above and other examples of implementations are also possible within the scope of the patent claims.

For example, the cardiovascular monitoring device can be fitted to an eyeglass, the optical and ICG-based CV sensors being placed on the eyeglass frame, for example, in the temple and/or nose regions while the ECG signal electrodes can be placed on each eyeglass branch. Here, a display unit using liquid crystal or the like can be placed in front of the eyeglass in order to display information related to, for example, heart rate to the user.

In another example, the optical and electrical CV sensors can be placed in a clip which is pinched to an earlobe or a portion of an ear, one clip on each ear being necessary for the ECG measurements.

In addition to the measured electrical PPG signal delivered by the receiver 6, other CV sensor signals, including the electric potentials measured at the ECG electrodes and/or the signal received by the pair of passive impedance electrodes 11, are transferred to the signal acquisition module 12 comprising, for example, an analog to digital (A/D) converter and signal filtering and shaping means.

The signal processing module 14 can be an adequately programmed digital signal processor or DSP or a general purpose microcontroller that also controls the control device 13 or a software module installed and executed by a processor. The cable 15 can be connected to the control device 13 through a USB connector or any other type of connector. The first and second ear located devices can be powered via the cable 15 by a battery (not shown) contained in the control device 13.

Other configurations are also possible, for example, a digital signal processor or DSP can be used in conjunction with signal filtration, A/D conversion, and other signal conditioning/processing within the module to extract useful heart rate information from the measured CV sensor signals. Alternatively, the functionalities of the signal acquisition module and signal processing module can be performed by using one or more application-specific integrated circuits (ASICs).

The signal processing module 14 may be housed within the ear located device 1, for example, within the hook 4. This configuration allows the wireless transmission of the sensor signals, for example, between the ear located device 1 and the control device 13. In the case of ECG and/ICG signals requiring a galvanic connection, only the processed signal can be the wirelessly transmitted. In the above configuration, the ear located device 1 can be powered with a battery (not shown) contained, for example, in the hook 4.

The ear located device 1 possibly also comprises a motion sensor 16 for measuring acceleration along three axes, for example, placed in the hook 4 as shown in Fig.1, but also possibly placed in the casing (2). The motion sensor 16 is preferably a MEMS-based three dimensional accelerometer as described in patent US7018338. It will however be appreciated that other types of accelerometers or motion detecting devices can be used provided they deliver a reliable measure of motion. For example, the motion sensor 16 could be a gyro-sensor of any suitable technology incorporating a one or multi-dimensional accelerometer, or a rotating or vibrating element.

The control device 10 may further comprise an output device 20 for outputting an indication of the detected pulse rate in the form of an optical, audible signal, or other sensorial signal. Such means could be a LED, a display, a buzzer, a vibrating device or any other suitable device adapted for transmitting information representative of the pulse rate measurement to the user. The outputted signal can also be superimposed on the sound signals produced by the media player and make it audible to the user through the sound transducer 3 of the ear located device 1. The outputted signal may also comprise an alarm signal when the estimated heart rate value reaches a determined threshold, which could be either a low or high threshold or both.

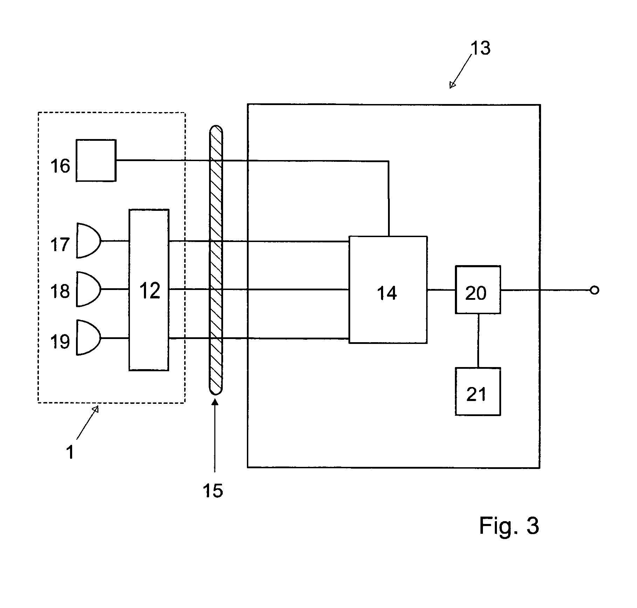

A schematic of the portable cardiovascular monitoring device of the invention is shown in Fig. 3. In the figure, the ear located device 1 represented by the dashed line comprises the PPG, ECG and ICG-based CV sensors schematically shown by the numerals 17, 18 and 19 respectively, the signal acquisition module 12 and the motion sensor 16. The control device 13 is also represented comprising the signal processing device 14, output device 20 and the memory 21 that is described below. Signals from the acquisition module 12 and motion sensor 16 are transferred to the signal processing module 14 through the cable 15.

The acquired signal originating from the PPG, ECG and/or ICG - based CV sensors are enhanced using the signal processing module 14 and possibly the motion reference signal provided by the motion sensor 16, in order to correct the acquired signals for movement artifacts. The signal enhancement is performed using a method described in PCT application PCT/EP2007/063469 by the present applicant.

The enhancement could also be performed exploiting the information contained in the motion reference signal. The method assumes that under rhythmical movements, artifacts on the acquired signal appear as harmonics of the movement frequency. The method comprises the two steps below.

In a first step, the fundamental movement frequency is extracted from the motion reference signal through one of the following technique: zero-crossing, parametric or non-parametric spectral estimation, autocorrelation, recurrence plots.

In a second step, the fundamental movement frequency extracted in the first step is used to enhance the acquired signal. Here, for example, a multiple notch filter can be employed to attenuate harmonics of movement contributions in the acquired signal. Alternatively, non-parametric enhancement can be used where the movement contribution is cancelled, for example, by putting high resolution restricted discrete Fourier transform estimations at zero from nearest lower minimum to nearest upper minimum at each harmonic of movement frequency.

The enhancement may also be performed by a subband approach. This may considerably increase the robustness of the subsequent heart rate estimation. In this case the acquired signal is filtered by typically three to four adjacent subband filters with a bandwidth of the fundamental movement frequency and located on harmonics of the movement frequency. Each subband filter is designed in such a way that the lower and upper corner frequency correspond to zeros of the given filter (as obtained for Tchebycheff class 2 filters or elliptic filters). The heart rate estimation may then be performed on each subband separately and the most reliable of all bands according to the reliability indexes may be retained.

The enhanced signals are further assessed for their reliability in order to select the most appropriate signal or merge the different reliable PPG, ECG and/or ICG CV signals and discard the unreliable signals. The reliability of the CV signals will depend on the measurement conditions such as reduced blood flow or/and low perfusion.

The reliability analysis can be based on the characteristics of CV signals and/ or associated heart rate estimations such as: the signal amplitude, pulse frequency, pulse frequency variations, and/or signal-to-noise ratio. Indeed, a signal having a too low pulse amplitude and/or a pulse frequency that is not plausible, i.e., too low, too high or too irregular, etc., may be determined as unreliable. The reliability analysis can also be performed based on or in combination with other parameters such as a low motion sensor signal, indicating that the user is at rest, or, conversely, a highly irregular motion sensor signal, indicating high possibility of shocks and/or non stationary accelerations, or a difference between the PPG signal, and ECG and/or ICG signals, etc.

The reliability assessment of the different CV sensor signals and associated heart rate estimations can be performed, for example, using a time-frequency distribution method such as Wavelet Transform (WT), Adaptive Wavetable Transforms (AWT) or the short term Fourier Transform (FT). Satistical assessement methods may also be employed (assessement of beat to beat variations in signal amplitudes and interbeat intervals). Other methods can also be used such as fuzzy logic or relying on a threshold level for comparison. These calculations are performed in the signal processing module 14.

In one embodiment, the reliability analysis is used to determine a reliability indicator that will take, for example, a value of "0" when, for example, the CV sensor signal amplitude is too low or the pulse frequency too irregular, and the CV sensor signal and/or associated heart rate estimation are determined as to be unreliable. Conversely, a reliability indicator of "1" will correspond to a signal that is determined as reliable. Intermediate values may also be used. For example, a value of 0.8 may indicate a high probability of having a reliable signal.

An estimation of a single robust user's heart rate can be obtained by combining the different heart rate estimations according to their reliability indicators or by selecting the heart rate estimations having the highest reliability indicator. For example, in conditions of reduced blood flow in the measured tissue, the amplitude of the measured PPG signal can be very low and unreliable, corresponding to a reliability indicator of "0". In these conditions, the ECG measured signal may not be good and reliable, corresponding to a reliability indicator value of "1" or near "1". Here, the and/or non stationary accelerations, or a difference between the PPG signal, and ECG and/or ICG signals, etc.

The reliability assessment of the different CV sensor signals and associated heart rate estimations can be performed, for example, using a time-frequency distribution method such as Wavelet Transform (WT), Adaptive Wavetable Transforms (AWT) or the short term Fourier Transform (FT). Satistical assessement methods may also be employed (assessement of beat to beat variations in signal amplitudes and interbeat intervals). Other methods can also be used such as fuzzy logic or relying on a threshold level for comparison. These calculations are performed in the signal processing module 14.

In one embodiment, the reliability analysis is used to determine a reliability indicator that will take, for example, a value of "0" when, for example, the CV sensor signal amplitude is too low or the pulse frequency too irregular, and the CV sensor signal and/or associated heart rate estimation are determined as to be unreliable. Conversely, a reliability indicator of "1" will correspond to a signal that is determined as reliable. Intermediate values may also be used. For example, a value of 0.8 may indicate a high probability of having a reliable signal.

An estimation of a single robust user's heart rate can be obtained by combining the different heart rate estimations according to their reliability indicators or by selecting the heart rate estimations having the highest reliability indicator. For example, in conditions of reduced blood flow in the measured tissue, the amplitude of the measured PPG signal can be very low and unreliable, corresponding to a reliability indicator of "0". In these conditions, the ECG measured signal may be good and reliable, corresponding to a reliability indicator value of "1" or near "1". Here, the user's heart rate will be estimated based on the ECG signal while the PPG signal will be discarded.

Alternatively, the heart rate estimations from the different CV sensor signals may be averaged, possibly with weights depending on their heart rate from signals from all CV sensors, or all CV sensors that currently deliver a reliable signal. The reliability of the CV sensor signals may also be estimated by using the short term variance and accepted range of values (statistical assessment using confidence interval assessment).

In order to improve power consumption of the portable cardiovascular monitoring device, the CV sensor which signal has been discarded can be switched off. In that case, the CV sensor will be switched on for appropriate short periods of time when performing the reliability analysis and switched off again, unless the latter reliability analysis determines the CV sensor signal to be reliable enough.

Alternatively to or in combination with the reliability analysis, the switching on and off of the CV sensor can be also controlled by the information provided by the motion sensor. For example, a motion sensor signal indicating that the user is at rest or is moving too irregularly may trigger the switching off of one or more CV sensors, based on the reliability analysis or in combination with it.

In the case all CV sensor signals are unreliable, the CV sensors can be switched off and the monitoring device is put in a stand-by mode. The monitoring device will be turned on again as soon as the reliability analysis described above determines at least one CV sensor signal to be reliable enough and/or when the motion sensor signal indicates that the user's motion intensity corresponds to at least one of the CV sensor functioning reliably.

In an aspect of the invention, a computer program product configured to be operable on the signal processing device 14 is provided in order to carry out the processing of the acquired signals described above, i.e., to correct the acquired signals for artifacts, perform the reliability analysis and control the CV sensor signal selection. The software product can be downloaded in a memory 21 associated with the signal processing device 14. The downloading operation can be performed using a storage reader device (not shown), such as a CD or DVD reader, a USB memory key or a flash memory, etc., integrated on the control device 13, or as an removable storage reader device (not shown), connected to the control device 13 through a USB connector or any other type of connector. The downloading operation can also be performed in a wireless fashion.

In a preferred embodiment of the invention, the artifact correction, reliability analysis and signal display are performed in real time on the stream of data delivered by the sensors. Post processing performed afterward, for example on a PC, is also possible.

The heart rate estimation can be used in combination with the signal processing module 14 to control other functionalities of the control device 13 such as, for example, the selection of a given music in function of the heart rate frequency. In addition to the heart rate, the software can be configured to prompt the user with other data such as level of exercise intensity, type of exercise to be undertaken, etc.

In addition to the heart rate, the different signals measured from the PPG, ECG and/or ICG-based CV sensors can also be used for the estimation of other physiologic parameters such as pulse oximetry, arterial pressure, etc.

### Reference numbers

- 1: First ear located device
- 2: Casing
- 3: Sound transducer
- 4: Hook
- 5: Optical emitter
- 6: Optical radiation receiver
- 7: Foam material
- 8: First ECG signal electrode
- 10: Pair of active impedance electrodes
- 11: Pair of passive impedance electrodes
- 12: Signal acquisition module
- 13: Control device
- 14: Signal processing device
- 15: Cable
- 16: Motion sensor
- 17: PPG-based CV sensor
- 18: ECG-based CV sensor
- 19: ICG-based CV sensor
- 20: Output device
- 21: Memory

## Claims

1. Portable cardiovascular monitoring device comprising a first cardiovascular (CV) sensor based on a photoplethysmography (PPG) technique for measuring a first CV sensor signal, a motion sensor (16) providing a motion reference signal, a signal processing module (14),
and at least a second CV sensor based on a different method for measuring at least a second CV sensor signal and processing the first and second CV sensor signals, and the motion reference signal, in the signal processing module (14) in order to obtain a single robust user's heart rate estimation; **characterized in that**
said monitoring device further comprises an ear located device (1), and **in that** the first and second CV sensors are comprised in said ear located device (1).

2. Portable cardiovascular monitoring device according claim 1,
wherein
said ear located device (1) comprises a casing (2) adapted to be placed in front of or inside the external auditory meatus of a user, the casing (2) comprising a sound transducer (3) being fastened to a hook (4).

3. Portable cardiovascular monitoring device according claim 2,
wherein
the first CV sensor comprises an optical emitter (5) and optical radiation receiver (6) placed in the hook (4) and/or the casing (2) in such a way as to allow the receiver (6) to pick up the portion of the optical radiation emitted by the emitter (5) that has passed through the tissue of the user's external ear.

4. Portable cardiovascular monitoring device according the claims 2 or 3, wherein
the second CV sensor is an electrocardiography (ECG) -based CV sensor comprising two signal electrodes, a first signal electrode placed in the hook (4) and/or the casing (2) of the first ear located device (1) and a second signal electrode placed in a hook and/or a casing of a second ear located device and worn on the user's opposite ear.

5. Portable cardiovascular monitoring device according the claims 2 or 3, wherein
the second CV sensor signal is an ICG-based CV sensor comprising a pair of active impedance electrodes (10) and a pair of passive impedance electrodes (11) being placed in the hook (4) and/or the casing (2) of the first ear located device (1) or placed in a hook and/or a casing of a second ear located device and worn on the user's opposite ear, in contact with the user's external ear skin.

6. Portable cardiovascular monitoring device according to any of the claims 1 to 5, comprising a third CV sensor based on ECG or ICG.

7. Portable cardiovascular monitoring device according to any of the claims 2 to 6, where the motion sensor (16) is placed in the hook (4) or in the casing (2) of the ear located device (1).

8. Portable cardiovascular monitoring device according to any of the claims 3 to 7, where the optical emitter (5) comprises two infrared light emitting devices emitting at two different wavelengths and where the receiver (6) comprises two pairs of photodiodes whose sensitivity ranges are adjusted to the respective wavelengths of the two infrared light emitting devices.

9. Method for determining a robust heart rate estimation of a user utilizing the portable cardiovascular monitoring device of claims 1 to 8, comprising
measuring the first CV sensor signal of the first photoplethysmography (PPG)-based CV sensor;
measuring the second CV signal of the second electrocardiography (ECG) or impedance cardiography (ICG)-based CV sensor; and
enhancing the first and second CV sensor signals using the motion reference signal, provided by the motion sensor (16), and the processing module (14) in order to remove noise and motion artifacts from said first and second CV sensor signals; **characterized by**
estimating the reliability of the enhanced CV sensor signal and determining a reliability indicator based on the estimated reliability; and
selecting at least one CV sensor signal or averaging the first and second CV sensor signals, according to a value of the reliability indicator for determining the robust user's heart rate estimation.

10. Method according to claim 9,
further comprising averaging the first CV sensor signal with the second CV sensor signal when the reliability of the first CV sensor signal has a value comprised between 1 and 0.8; and selecting the second CV sensor signal when the reliability of the first CV sensor signal has a value below 0.8.

11. Method according to the claims 9 or 10, wherein
said estimating the reliability is based on characteristics of CV signals in combination with the signal provided by the motion sensor signal.

12. Method according to any of the claims 9 to 11, where the unreliable CV sensors are switched off.

13. Method according to any of the claims 9 to 12, where the most likely temporal position of at least one heart pulse is estimated based on CV signals from several sensors.

14. Computer carrier comprising program code portions to be executed by a signal processing device (14), said program code portions being configured to carry out the method of one of the claims 9 to 13 when said program is executed by said signal processing device (14).

## Patentansprüche

1. Tragbares kardiovaskuläres Überwachungsgerät umfassend einen ersten Kardiovaskulären (CV) Sensor, der basierend auf einer photoplethysmographie (PPG) Technik basiert, um ein erstes CV Sensorsignal zu messen, einen Bewegungssensor (16), der ein Bewegungsreferenzsignal bereitstellt, ein Signalverarbeitungsmodul (14), und mindestens einen zweiten CV Sensor, der auf einem verschiedenen Verfahren for die Messung mindestens eines zweiten CV Sensorsignal basiert, und Verarbeiten des ersten und zweiten CV Sensorsignals und des Bewegungsreferenzsignals in dem Signalverarbeitungsmodul (14), um eine einzelne robuste Herzfrequenzschätzung des Benutzers zu bestimmen; **gekennzeichnet, dadurch dass**
besagte Überwachungsvorrichtung weiter ein sich am Ohr befindliche Vorrichtung (1) umfasst, und **dadurch**, dass sich die ersten und zweiten Sensoren in besagter, sich am Ohr befindenden Vorrichtung (1) befinden.

2. Tragbares kardiovaskuläres Überwachungsgerät gemäss Anspruch 1, wobei besagte, sich am Ohr befindende Vorrichtung (1) ein Gehäuse (2) umfasst, das angepasst ist, vor oder in dem externen Gehörgang des Benutzers platziert zu werden, das Gehäuse (2) umfasst einen Soundwandler (3), der an einen Haken (4) befestigt wird.

3. Tragbares kardiavaskuläres Überwachungsgerät gemäss Anspruch 2, wobei der erste CV Sensor einen optischen Emitter (5) und ein optischen Strahlungsempfänger (6) umfasst, die in dem Haken (4) und/oder dem Gehäuse (2) derart platziert sind, dass dem Empfänger (6) erlaubt wird, das Teil der optischen Strahlung aufzunehmen, die durch den Emitter (5) emittiert wird und das Gewebe des externen Ohrs des Benutzers durchquert hat.

4. Tragbares kardiovaskuläres Überwachungsgerät gemäss dem Anspruch oder 3, wobei der zweite CV Sensor ein elektrokardiogramm (ECG)-basierter CV Sensor ist, der zwei Signalelektroden umfasst, eine erste Signalelektrode, die in dem Haken (4) und/oder dem Gehäuse (2) der ersten, sich am Ohr befindenden Vorrichtung (1) und einer zweiten Signalelektrode, die in einem Haken und/oder einem Gehäuse einer zweiten, sich am Ohr befindenden Vorrichtung befindet, die am gegenüberliegenden Ohr des Benutzers getragen wird.

5. Tragbares kardiovaskuläres Überwachungsgerät gemäss dem Anspruch 2 oder 3, wobei das zweite CV Sensorsignal ein ICG-basierter CV Sensor ist, der ein Paar von aktiven Impedanzelektroden (10) und ein Paar von passiven Impedanzelektroden (11) umfasst, die sich in dem Haken (4) und/oder dem Gehäuse (2) der ersten, sich am Ohr befindenden Vorrichtung (1) oder in einem Haken und/oder einem Gehäuse einem Gehäuse einer zweiten, sich am Ohr befindenden Vorrichtung, die am gegenüberliegenden Ohr des Benutzers getragen wird, befinden, in Kontakt mit der externen Haut des Ohrs.

6. Tragbares kardiovaskuläres Überwachungsgerät gemäss einem der Ansprüche 1 bis 5, umfassend einen dritten CV Sensor, der auf ECG oder ICG basiert.

7. Tragbares kardiovaskuläres Überwachungsgerät gemäss einem der Ansprüche 2 bis 6, in welchem der Bewegungssensor (16) in dem Haken (4) oder in dem Gehäuse (2) der sich am Ohr befindenden Vorrichtung (1) platziert ist.

8. Tragbares kardiovaskuläres Überwachungsgerät gemäss einem der Ansprüche 3 bis 7, in welchem der optische Emitter (5) zwei infrarote Licht emittierende Vorrichtungen umfasst, die an zwei verschiedenen Wellenlängen emittieren, und in welchem der Empfänger (6) zwei Paare von Photodioden umfasst, deren Sensibilitätsbereiche an die jeweilige Wellenlänge der zwei Infraroten Licht emittierenden Vorrichtungen angepasst sind.

9. Verfahren, um eine robuste Herzfrequenzschätzung eines Benutzers unter Verwendung der tragbaren kardiovaskulären Überwachungsvorrichtung der Ansprüche 1 bis 8 zu bestimmen, umfassend Messen des ersten CV Sensorsignals des ersten photaplethysmographie (PPG)-basierten Sensors;
Messen des zweiten CV Signals des zweiten elektrokardiogramm (ECG) oder impedanzcardiogramm (ICG)-basierten Sensors; und
Verbessern des ersten und des zweiten CV Sensorsignais unter Verwendung des Bewegungsreferenzsignals, das durch den Bewegungssensor (16) und des Verarbeitungsmoduls (14) bereitgestellt wird, um das Rauschen und die Bewegungsartefakte von besagtem ersten und zweiten CV Sensorsignalen zu entfernten; **gekennzeichnet durch** Schätzen der Zuverlässigkeit des verbesserten CV Sensorsignals und Bestimmen eines Zuverlässigkeitsindikators, der auf der geschätzten Zuverlässigkeit basiert; und
Auswahl mindestens eines CV Sensorsignals oder Bilden des Durchschnittswerts des erstens und zweiten CV Sensorsignals gemäss einem Wert des Zuverlässigkeitsindikators, um die robuste Herzfrequenzschätzung des Benutzers zu bestimmen.

10. Verfahren gemäss Anspruch 9, weiter umfassend Bilden des Durchschnittswerts des ersten CV Sensors mit dem zweiten CV Sensorsignal, wenn die Zuverlässigkeit des ersten CV Sensors einen Wert zwischen 1 und 0.8 umfasst; und Auswahl des zweiten CV Sensorsignals, wenn die Zuverlässigkeit des ersten CV Sensorsignals einen Wert unter 0.8 einnimmt.

11. Verfahren gemäss einem der Ansprüche 9 oder 10, in welchem besagtes Schätzen der Verlässlichkeit auf Charakteristiken der CV Signale in Kombination mit dem Signal basiert, das durch den Bewegungssensorsignal bereitgestellt wird.

12. Verfahren gemäss einem der Ansprüche 9 bis 11, in welchem die unzuverlässigen CV Sensoren abgeschaltet werden.

13. Verfahren gemäss einem der Ansprüche 9 bis 12, in welchem die wahrscheinlichste zeitliche Position von mindestens einem Herzpuls auf der Basis von CV Signalen von verschiedenen Sensoren geschätzt wird.

14. Computerträger umfassend Programmcodeteile, die durch eine Signalverarbeitungsvorrichtung (14) ausgeführt werden, besagte Programmcodeteile sind konfiguriert, um das Verfahren nach einem der Ansprüche 9 bis 13 auszuführen, wenn besagtes Programm durch besagte Signalverarbeitungsvorrichtung (14) ausgeführt wird.

## Revendications

1. Dispositif de surveillance cardio-vascuiaire portable comprenant un premier capteur cardio-vasculaire (CV) basé sur une technique de photoplethysmographie (PPG), pour mesurer un premier signal de capteur CV, un capteur de mouvement (16), qui fournit un signal de mouvement de référence, un module de traitement de signal (14), et au moins un second capteur CV basé sur une méthode différente pour mesurer au moins un second signal de capteur CV, et traiter les premier et second signaux de capteurs CV et le signal de mouvement de référence dans le module de traitement du signal (14) pour obtenir une seule estimation fiable de la fréquence cardiaque d'un utilisateur;
**caractérisé en ce que**
ledit dispositif de surveillance comporte d'autre part un dispositif situé au niveau de l'oreille (1), et **en ce que** le premier et le second capteurs CV sont compris dans ledit dispositif situé au niveau de l'oreille (1).

2. Dispositif de surveillance cardio-vasculaire portable selon la revendication 1, selon lequel
ledit dispositif situé au niveau de l'oreille (1) comprend un boîtier (2) adapté pour être placé devant ou à l'intérieur du conduit auditif externe d'un utilisateur, le boîtier (2) comprenant un transducteur acoustique (3) attaché à un crochet (4).

3. Dispositif de surveillance cardio-vasculaire portable selon la revendication 2, selon lequel
le premier capteur CV comprend un émetteur optique (5) et un récepteur de rayonnement optique (6) placés dans le crochet (4) et/ou le boîtier (2), de manière à permettre au récepteur (6) de capter la portion du rayonnement optique émis par l'émetteur (5) qui a traversé le tissu de l'oreille externe de l'utilisateur,

4. Dispositif de surveillance cardio-vasculaire portable selon la revendication 2 ou 3, selon lequel
le second capteur CV est un capteur basé sur une technique d'electrocardiographie (ECG) comprenant deux électrodes de signal, la première électrode de signal étant placée dans le crochet (4) et/ou le boîtier (2) du premier dispositif situé au niveau de l'oreille (1) et la seconde électrode de signal étant placée dans le crochet et/ou le boîtier d'un second dispositif situé au niveau de l'oreille et porté sur l'oreille opposée de l'utilisateur.

5. Dispositif de surveillance cardio-vasculaire portable selon la revendication 2 ou 3, selon lequel
le second capteur CV est un capteur basé sur une technique ICG comprenant une paire d'électrodes actives d'impédance (10) et une paire d'électrodes passives d'impédance (11) placées dans le crochet (4) et/ou le boîtier (2) du premier dispositif situé au niveau de l'oreille (1) ou placées dans un crochet et/ou un boîtier d'un second dispositif situé au niveau de l'oreille, et porté sur l'oreille opposée de l'utilisateur, en contact avec la peau de l'oreille externe de l'utilisateur.

6. Dispositif de surveillance cardio-vasculaire portable selon l'une des revendications 1 a 5, comprenant un troisième capteur CV basé sur une technique ECG ou ICG.

7. Dispositif de surveillance cardio-vasculaire portable selon l'une des revendications 2 à 6, dans lequel le capteur de mouvement (16) est placè dans le crochet (4) ou dans le boîtier (2) du dispositif situé au niveau de l'oreille (1).

8. Dispositif de surveillance cardio-vasculaire portable selon l'une des revendications 3 à 7, dans lequel l'émetteur optique (5) comprend deux dispositifs émetteurs de lumière infrarouge émettant à deux longueurs d'onde différentes, et dans lequel le récepteur (6) comprend deux paires de photodiodes dont les plages de sensibilité sont ajustées respectivement aux longueurs d'ondes des deux dispositifs émetteurs de lumière infrarouge.

9. Méthode pour obtenir une estimation fiable de la fréquence cardiaque d'un utilisateur par l'utilisation du dispositif de surveillance cardio-vascuiaire portable selon l'une des revendications 1 à 8, comprenant la mesure d'un premier signal de capteur CV du premier capteur CV basé sur une technique de photoplethysmographie (PPG);
la mesure d'un second signal de capteur CV du second capteur basé sur une technique d'électrocardiographie (ECG) ou de cardiographie d'impédance (ICG); et
l'amélioration des premier et second signaux de capteurs CV par l'utilisation du signal de mouvement de référence fourni par le capteur de mouvement (16) et le module de traitement de signal (14), de manière à supprimer les artéfacts de bruit et de mouvement desdits premier et second signaux de capteurs CV; **caractérisée par** l'estimation de la fiabilité du signal amélioré de capteur CV et la détermination d'un indicateur de fiabilité basé sur la fiabilité estimée; et la sélection d'au moins un signal de capteur CV ou le calcul d'une moyenne des premier et second signaux de capteurs CV, en fonction d'une valeur de l'indicateur de fiabilité pour obtenir l'estimation fiable de la fréquence cardiaque de l'utilisateur.

10. Méthode selon la revendication 9,
comprenant d'autre part le calcul de la moyenne entre le premier signal de capteur CV et le second signal de capteur CV lorsque la fiabilité du premier signal de capteur CV comprend une valeur entre 1 et 0.8; et la sélection du second signal de capteur CV, lorsque la fiabilité du premier signal de capteur CV comprend une valeur inférieure à 0.8.

11. Méthode selon l'une des revendications 9 ou 10, selon laquelle ladite estimation de la fiabilité est basée sur des caractéristiques des signaux CV en combinaison avec le signal fourni par le signal de capteur de mouvement.

12. Méthode selon l'une des revendications 9 à 11, dans laquelle les capteurs C.V non fiables sont déconnectés.

13. Méthode selon l'une des revendications 9 à 12, dans laquelle la position temporale la plus probable d'au moins une pulsation cardiaque est estimée en se basant sur les signaux CV de plusieurs capteurs.

14. Support informatique comprenant des portions de code programme à faire exécuter par un module de traitement de signal (14), lesdites portions de code programme étant configurées pour effectuer la méthode selon l'une des revendications 9 à 13 lorsque ledit programme est exécuté par ledit module de traitement de signal (14).
